# EUROPÄISCHE PATENTSCHRIFT

(11) **EP 2 991 720 B1**
(45) Veröffentlichungstag und Bekanntmachung des Hinweises auf die Patenterteilung: **12.04.2017**
(21) Anmeldenummer: 14705806.9
(22) Anmeldetag: 24.02.2014
(51) Int. Cl.: A61M 39/12, A61M 39/02, A61M 39/04

(54) **PORT FÜR EINEN KATHETER**
PORT FOR A CATHETER
PORT POUR UN CATHÉTER

(30) Priorität: 03.05.2013 EP 13166447
(43) Veröffentlichungstag der Anmeldung: 09.03.2016
(73) Patentinhaber: Fresenius Kabi Deutschland GmbH, 61352 Bad Homburg (DE)
(72) Erfinder: JOCHUM, Christoph, 61130 Nidderau (DE); PAPIOREK, Martina, 65597 Hünfelden 1 (DE); SCHUMACHER, Rainer, 65232 Taunusstein (DE); HAINDL, Hans, 30974 Wennigsen (DE); KÄMEREIT, Markus, 48301 Nottuln (DE); KLEVE, Klaus, 48231 Warendorf (DE)
(74) Vertreter: Kusche, Robert
(86) Internationale Anmeldenummer: PCT/EP2014/053552
(87) Internationale Veröffentlichungsnummer: WO 2014/177298

(56) Entgegenhaltungen:
- EP-A1- 2 561 899
- EP-B1- 1 675 641
- US-A- 4 723 948
- US-A- 5 718 682
- US-A1- 2008 319 421
- US-A1- 2011 160 673
- US-A1- 2012 109 068

## Beschreibung

Die Erfindung betrifft einen Port für einen Katheter nach dem Oberbegriff des Anspruchs 1.

Ein derartiger Port umfasst eine Kammer zum Aufnehmen eines medizinischen Wirkstoffs, ein Anschlussstück zum Anschließen eines Katheters, um eine Strömungsverbindung zwischen der Kammer und dem Katheter herzustellen, und mindestens einen Hebel, der um eine Schwenkachse eines Gehäuses schwenkbar gelagert ist. Der mindestens eine Hebel ist ausgebildet, in einer ersten Schwenkstellung ein Ansetzen des Katheters an das Anschlussstück zuzulassen und in einer zweiten Schwenkstellung den Katheter klemmend an dem Anschlussstück zu halten.

Ein derartiger Port, wie er beispielsweise aus der EP 1 675 641 B1 bekannt ist, kann einem Patienten implantiert werden, indem er beispielsweise unter die Haut eines Patienten (subkutan) eingebracht und dort befestigt wird. Der Port dient dann zur Infusion von Medikamenten, Blutprodukten, Nährstoffen oder anderen medizinischen Wirkstoffen in das venöse oder arterielle System eines Patienten. Mittels des Ports kann ein Wirkstoff einem Patienten insbesondere wiederholt über einen längeren Zeitraum verabreicht werden. Weil der Port dabei vollständig unter die Haut eines Patienten implantiert werden kann, ist das Risiko für Infektionen reduziert, und Wirkstoffe können über einen längeren Zeitraum gezielt zur Behandlung eines Patienten verabreicht werden, ohne dass dazu der Patient stationär in ein Krankenhaus aufgenommen werden muss und ohne dass der Port den Patienten im Alltag wesentlich behindert.

Bei einer Infusion wird ein Wirkstoff aus der im Gehäuse eingefassten Kammer über einen mit dem Anschlussstück des Ports verbundenen Katheter abgeleitet und dem Patienten, beispielsweise dem venösen oder arteriellen System des Patienten, zugeführt. Der Katheter ist hierbei zusammen mit dem Port in dem Patienten implantiert und derart verlegt, dass der Wirkstoff an einen vorbestimmten Ort im Patienten transportiert wird.

Bei einem solchen Port ist dafür Sorge zu tragen, dass der an das Anschlussstück angesetzte Katheter bei implantiertem Port nicht von dem Anschlussstück rutschen und sich somit von dem Port lösen kann. Hierzu weist der in der EP 1 675 641 B1 beschriebene Port Klemmbacken auf, die in einer ersten Stellung von dem Gehäuse des Ports abstehen und zum klemmenden Halten des Katheters an dem Gehäuse in eine zweite Stellung überführt werden können, in der sie den Katheter klemmend zwischen sich aufnehmen.

Aus der US 4,723,948 ist ein Port bekannt, bei dem ein Katheter mittels einer Hülse an ein Anschlussstück des Ports angeschlossen werden kann. Die Hülse besteht aus zwei Teilen, die über ein Filmscharnier miteinander verbunden sind und zum formschlüssigen Verbinden des Katheters mit dem Anschlussstück in einer geschlossenen Stellung der Hülse das Anschlussstück umschließen.

Es besteht ein Bedürfnis nach einem Port, der ein leichtes Ansetzen eines Katheters an ein Anschlussstück ermöglicht und auf einfache, leicht zu handhabende Weise einen zuverlässigen Halt des Katheters an dem Anschlussstück bereitstellen kann. Dabei ist zu berücksichtigen, dass sich die Handhabung eines solchen Ports häufig dadurch erschweren kann, dass ein Nutzer, beispielsweise ein Arzt bei einer Operation, oft Handschuhe trägt, so dass die Handhabung entsprechend einfach und ohne großen Kraftaufwand möglich sein sollte.

Aufgabe der vorliegenden Erfindung ist es, einen Port für einen Katheter bereitzustellen, der bei einfacher Handhabung einen zuverlässigen Halt eines Katheters am Port ermöglicht.

Diese Aufgabe wird durch einen Gegenstand mit den Merkmalen des Anspruchs 1 gelöst.

Demnach ist vorgesehen, dass der mindestens eine Hebel einen ersten Hebelabschnitt und einen von dem ersten Hebelabschnitt unterschiedlichen, zweiten Hebelabschnitt aufweist. Der erste Hebelabschnitt trägt eine Rasteinrichtung zum Verrasten des mindestens einen Hebels mit dem Gehäuse und/oder einem ersten Hebelabschnitt eines weiteren Hebels in der zweiten Schwenkstellung, und der zweite Hebelabschnitt trägt eine Klemmeinrichtung zum klemmenden Halten des Katheters in der zweiten Schwenkstellung.

Die vorliegende Erfindung geht von dem Gedanken aus, zur Fixierung des Katheters an dem Anschlussstück einen oder mehrere Hebel vorzusehen, die jeweils über zwei Hebelabschnitte verfügen. Ein erster Hebelabschnitt trägt hierbei eine Rasteinrichtung, während ein zweiter Hebelabschnitt eine Klemmeinrichtung aufweist. Die Rasteinrichtung dient dazu, den Hebel in seiner zweiten Stellung, in der er ausgebildet ist, einen Katheter klemmend an dem Anschlussstück des Ports zu halten, mit dem Gehäuse des Ports zu verrasten und/oder durch Verrastung mit einem ersten Hebelabschnitt eines weiteren Hebels an dem Gehäuse zu fixieren, so dass der Hebel nicht ohne weiteres, zumindest nicht ohne Lösen der Verrastung, aus seiner zweiten Stellung herausgeschwenkt werden kann. Der klemmende Halt des Katheters an dem Anschlussstück wird hierbei über die Klemmeinrichtung bereitgestellt, die klemmend auf den Katheter einwirkt und in der zweiten Stellung somit den Katheter sicher und zuverlässig an dem Port hält.

In der ersten Stellung des mindestens einen Hebels kann der Katheter an den Port angesetzt werden, so dass der Katheter somit z.B. vor Implantieren des Ports mit dem Port verbunden werden kann. In dieser ersten Stellung ist ein Klemmabschnitt der Klemmeinrichtung derart von dem Anschlussstück entfernt, dass der Katheter an das Anschlussstück angesetzt, beispielsweise auf das Anschlussstück aufgeschoben werden kann, ohne dass dies durch den Klemmabschnitt des zweiten Hebelabschnitts des Hebels behindert wird. Beim Überführen in die zweite Schwenkstellung wird durch Verschwenken des Hebels dann der Klemmabschnitt dem Katheter angenähert, so dass der Katheter zwischen dem Klemmabschnitt und dem Anschlussstück verklemmt und dadurch an dem Port arretiert wird.

Vorzugsweise ist der zweite Hebelabschnitt kürzer als der erste Hebelabschnitt. Der erste Hebelabschnitt kann beispielsweise in der ersten Stellung des Hebels von dem Gehäuse abstehen und zum Überführen des Hebels in seine zweite Stellung manuell betätigt werden, indem er hin zum Gehäuse verschwenkt wird. Dadurch, dass der erste Hebelabschnitt länger als der zweite Hebelabschnitt ist, ergibt sich ein vorteilhaftes Hebelverhältnis, das ein Verschwenken des Hebels mit vergleichsweise geringer Betätigungskraft ermöglicht, dabei aber an dem zweiten Hebelabschnitt eine vergleichsweise große Klemmkraft zum klemmenden Halten des Katheters an dem Anschlussstück bereitstellt.

Grundsätzlich können der erste Hebelabschnitt und der zweite Hebelabschnitt Bestandteil desselben Hebelarms sein, wobei der zweite Hebelabschnitt in seiner Hebellänge jedoch unterschiedlich, vorzugsweise kürzer als der erste Hebelabschnitt ist. Vorzugsweise erstrecken sich der erste Hebelabschnitt und der zweite Hebelabschnitt aber in unterschiedliche Richtungen von der Schwenkachse, indem sich der erste Hebelabschnitt beispielsweise in eine erste Richtung von der Schwenkachse und der zweite Hebelabschnitt in eine zweite, der ersten Richtung entgegengesetzte Richtung von der Schwenkachse erstrecken.

An dem Klemmabschnitt des zweiten Hebelabschnitts ist vorteilhafterweise mindestens eine Klemmrippe angeordnet, die sich quer, beispielsweise senkrecht, zu einer Schwenkrichtung, entlang derer der mindestens eine Hebel zum Überführen von seiner ersten Stellung in die zweite Stellung verschwenkt wird, erstreckt. Durch solche quer erstreckten Klemmrippen, die bei Überführen des Hebels in seine zweite Stellung dem an das Anschlussstück angesetzten Katheter angenähert werden, wird eine besonders feste Klemmung des Katheters an dem Anschlussstück bereitgestellt, die einen zuverlässigen Halt des Katheters an dem Port gewährleistet.

Die an dem ersten Hebelabschnitt ausgebildete Rasteinrichtung kann beispielsweise einen Rasthaken aufweisen, der in der zweiten Schwenkstellung, also bei an dem Port arretierten Katheter, rastend mit einem zugeordneten Rastvorsprung des Gehäuses und/oder eines weiteren Hebels in Eingriff steht. Dadurch, dass die Rasteinrichtung an dem vorzugsweise längeren ersten Hebelabschnitt des Hebels angeordnet ist, kann eine vergleichsweise geringe Rastkraft zum sicheren Arretieren des Hebels in seiner zweiten Stellung ausreichen. Dadurch, dass zudem die Rasteinrichtung räumlich getrennt ist von der Klemmeinrichtung, kann gewährleistet werden, dass beim Überführen des Hebels in seine zweite Stellung die Rasteinrichtung hörbar einschnappt, so dass ein Nutzer eine Rückmeldung über das Erreichen der zweiten Schwenkstellung des Hebels und somit das Erreichen der Klemmposition erhält. Dadurch, dass die Rasteinrichtung räumlich getrennt ist von der Klemmeinrichtung und somit von dem Katheter, kann eine Geräuschbildung beim Einrasten nicht gedämpft werden, so dass ein Nutzer ein haptisch und/oder akustisch wahrnehmbares und vorzugsweise auch angenehmes Feedback erfährt.

Grundsätzlich dient die Verrastung dazu, den mindestens einen Hebel sicher in seiner zweiten Schwenkstellung zu halten, um insbesondere bei implantiertem Port ein Ausschwenken des Hebels - das mit einem Lösen der Arretierung des Katheters am Port einhergehen würde - zu vermeiden. Dabei kann vorgesehen sein, die Verrastung des mindestens einen Hebels mit dem Gehäuse oder einem anderen Hebel lösbar auszugestalten, indem beispielsweise an einem Gehäuseboden des Gehäuses eine Betätigungsöffnung vorgesehen ist, durch die hindurch auf die Rasteinrichtung beispielsweise mittels eines geeigneten Formstücks zugegriffen werden kann, um die Rasthaken der Rasteinrichtung aus ihrem Formschluss mit den Rastvorsprüngen des Gehäuses und/oder des weiteren Hebels zu lösen.

In einer vorteilhaften Ausgestaltung weist der Port zwei Hebel auf, die jeweils einen ersten Hebelabschnitt und einen von dem ersten Hebelabschnitt unterschiedlichen, zweiten Hebelabschnitt aufweisen. Die Hebel sind hierbei vorzugsweise um im Wesentlichen gleichgerichtete, insbesondere parallel zueinander erstreckte, Schwenkachsen verschwenkbar, wobei sich die Schwenkachsen vorzugsweise zumindest näherungsweise quer, insbesondere senkrecht, zu einer Erstreckungsrichtung des als Kanüle ausgebildeten Anschlussstücks erstrecken. Durch Verschwenken der Hebel in ihre jeweils zweite Schwenkstellung kann somit ein auf das Anschlussstück aufgesteckter Katheter beidseits zwischen den Hebeln und dem Anschlussstück verklemmt werden, so dass ein besonders sicherer Halt des Katheters an dem Port gewährleistet ist.

In der zweiten Schwenkstellung ist der Katheter an dem Port arretiert. Nehmen die Hebel hingegen die erste Schwenkstellung ein, so sind die Klemmabschnitte an den zweiten Hebelabschnitten der Hebel von dem Anschlussstück entfernt, so dass der Katheter auf das Anschlussstück aufgeschoben werden kann. Die Hebel bilden hierbei zwischen ihren zweiten Hebelabschnitten vorzugsweise eine Durchführungsöffnung aus, indem sie geeignete Aussparungen aufweisen, durch die hindurch der Katheter zum Ansetzen an das Anschlussstück in der ersten Schwenkstellung der Hebel geführt werden kann.

Die Hebel müssen nicht notwendigerweise mit dem Gehäuse verrastet werden. Denkbar und möglich ist auch, die Rasteinrichtungen der zwei Hebel so auszugestalten, dass die zwei Hebel miteinander verrasten können und sich so gegenseitig in der zweiten, eingeschwenkten Stellung halten.

Um die Hebel aus ihrer ersten Stellung zum Arretieren eines an das Anschlussstück angesetzten Katheters in die zweite Stellung zu überführen, müssen die Hebel verschwenkt werden. Um dabei sicherzustellen, dass die Verschwenkbewegung der Hebel gleichförmig erfolgt, können die Hebel jeweils eine Verzahnung an ihrem zweiten Hebelabschnitt aufweisen, wobei die Verzahnungen der Hebel derart miteinander in Eingriff stehen, dass bei einem Verschwenken des einen Hebels auch der andere Hebel in synchroner Weise mit verschwenkt wird. Ein Verschwenken der Hebel ist damit nur synchron möglich, so dass die Klemmabschnitte der Klemmeinrichtung der beiden Hebel dem Katheter in gleichförmiger Weise angenähert werden und die Klemmung des Katheters zwischen den Hebeln und dem Anschlussstück somit in synchroner Weise hergestellt wird.

Beim Überführen des mindestens einen Hebels in seine zweite Stellung wird der Hebel vorzugsweise dem Gehäuse angenähert. An dem Gehäuse kann hierbei mindestens ein Druckpunkt in Form einer Auswölbung angeordnet sein, der beim Überführen des mindestens einen Hebels von der ersten Schwenkstellung in die zweite Schwenkstellung mit einer Vertiefung an dem mindestens einen Hebel in Eingriff zu bringen ist, wobei zum In-Eingriff-Bringen des Druckpunkts am Gehäuse mit der Vertiefung am Hebel ein gewisser, vorbestimmter Kraftaufwand erforderlich ist. Auf diese Weise kann sichergestellt werden, dass ein Verschwenken des mindestens einen Hebels in seine zweite Schwenkstellung nur in bewusster Weise erfolgen kann, indem ein Nutzer beim Verschwenken des Hebels in seine zweite Schwenkstellung aufgrund des Druckpunktes einen Widerstand erfährt, den er überwinden muss, um den Hebel in die zweite Schwenkstellung zu bringen.

In einer alternativen Ausführungsform ist der Hebel ohne Vertiefung ausgestattet. Der Druckpunkt wird beim Vorliegen eines gewissen, vorbestimmten Kraftaufwands überwunden, ohne dass ein "In-Eingriff-Bringen" stattfindet.

Beispielsweise an einer Bodenplatte des Gehäuses kann zudem eine Sichtöffnung vorgesehen sein, durch die hindurch visuell inspiziert werden kann, ob ein Katheter richtig an das Anschlussstück angesetzt und an dem Anschlussstück gehalten ist. Die Sichtöffnung erlaubt einen Blick auf das Anschlussstück, so dass auch bei hergestellter Klemmverbindung geprüft werden kann, ob der Katheter in gewünschter Weise mit dem Port verbunden ist.

Der Port weist vorzugsweise eine an dem Gehäuse gehaltene Membran auf, die mittels einer Punktionskanüle durchstochen werden kann, um einen Wirkstoff in die Kammer des Gehäuses einzuspritzen. Um hierbei zu verhindern, dass eine Punktionskanüle bei dem Versuch, die Membran zu punktieren, von dem Gehäuse abgleiten und hin zu dem mit dem Port verbundenen Katheter gelangen kann - was potentiell zu einer Beschädigung des Katheters führen könnte -, weist das Gehäuse ein Ableitelement (auch als "Prallkante" bezeichnet) auf, das an einer hin zu dem Anschlussstück weisenden Seite der Membran angeordnet ist und ein Abrutschen einer Punktionskanüle hin zu dem an das Anschlussstück des Ports angeschlossenen Katheter verhindert. Das Ableitelement kann beispielsweise Bestandteil einer an dem Gehäuse verlaufenden nutförmigen Vertiefung sein, die bei einem Abgleiten einer Punktionskanüle die Punktionskanüle weg von dem Katheter führt.

Der der Erfindung zugrundeliegende Gedanke soll nachfolgend anhand der in den Figuren dargestellten Ausführungsbeispiele näher erläutert werden. Es zeigen:
- Fig. .1: eine perspektivische Explosionsansicht eines Ports mit einem daran anzuschließenden Katheter;
- Fig. 2: eine perspektivische Ansicht des Ports mit dem daran anzuschließenden Katheter, vor dem Anschließen des Katheters;
- Fig. 3: eine perspektivische Ansicht des Ports mit daran angeschlossenem Katheter;
- Fig. 4A: eine Seitenansicht des Ports mit dem daran anzuschließenden Katheter, vor dem Anschließen des Katheters;
- Fig. 4B: eine Schnittansicht entlang der Linie A - A gemäß Fig. 4A;
- Fig. 5A: eine Seitenansicht des Ports mit daran angeschlossenem Katheter;
- Fig. 5B: eine Schnittansicht entlang der Linie A - A gemäß Fig. 5A;
- Fig. 6: eine perspektivische Ansicht des Ports mit daran angeschlossenem Katheter, schräg von unten;
- Fig. 7A: eine Seitenansicht des Ports mit daran anzuschließendem Katheter, vor dem Anschließen des Katheters; und
- Fig. 7B: eine Schnittansicht entlang der Linie A - A gemäß Fig. 7A.

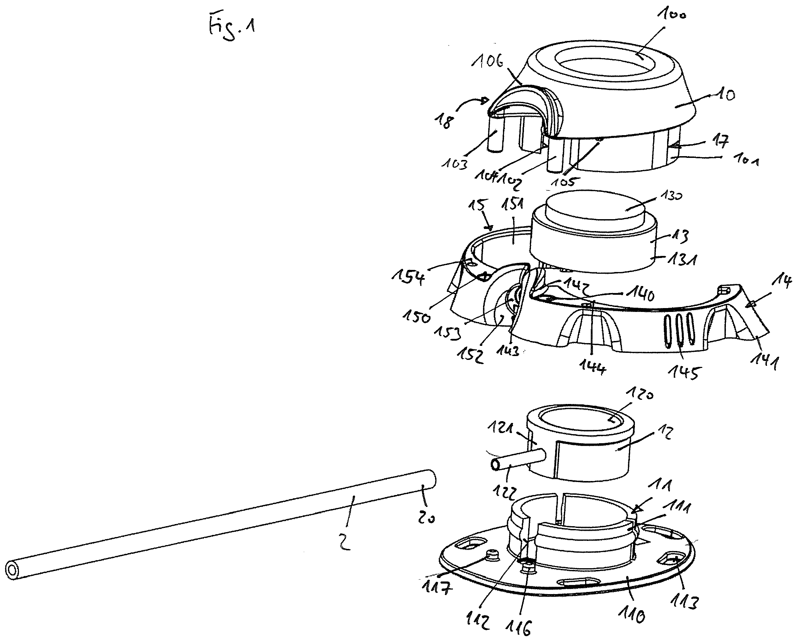

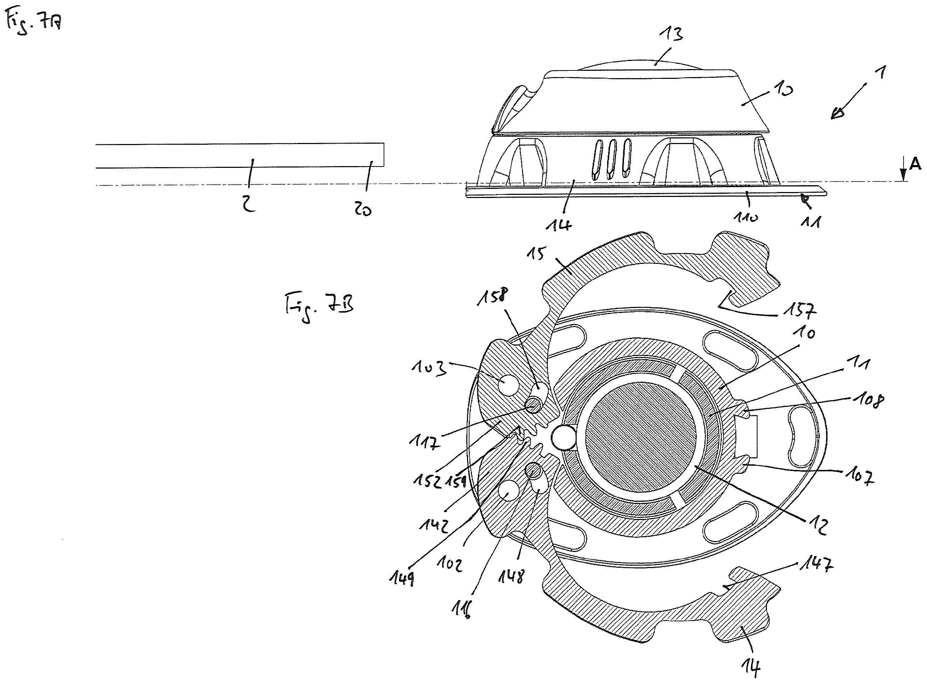

Fig. 1 bis 7A, 7B zeigen ein Ausführungsbeispiels eines Ports 1, an dem ein Katheter 2 angeschlossen werden kann. Der Port 1 weist ein durch Gehäuseteile 10, 11 gebildetes Gehäuse auf, das eine in einem Einsatzteil 12 gebildete Kammer 120 einfasst.

Der Port 1 kann zusammen mit einem daran angeschlossenen Katheter 2 in einen Patienten implantiert werden. Ein in der Kammer 120 befindlicher Wirkstoff kann dann über den Katheter 2 an einen Wirkort in dem Patienten verbracht werden, um auf diese Weise dem Patienten einen Wirkstoff, beispielsweise ein Medikament, Nährstoffe oder eine andere medizinische Flüssigkeiten, zuzuführen.

Der Port 1 weist eine Membran 13 auf, die im montierten Zustand des Ports 1 mit einem Kopfabschnitt 130 in eine Öffnung 100 des oberen Gehäuseteils 10 eingesetzt ist und die Kammer 120 des Einsatzteils 12 nach oben hin abschließt. Die Membran 13 kann durch eine Punktionskanüle punktiert werden, um auf diese Weise einen Wirkstoff in die Kammer 120 einzuspritzen und somit den Port 1 zu befüllen.

Das untere Gehäuseteil 11 weist eine Bodenplatte 110 mit Nahtlöchern 113 zum Befestigen des Port 1 in einem Patienten und einen Steckabschnitt 111, der in eine Einfassung 101 des oberen Gehäuseteils 10 einzustecken ist, auf. Zur Montage des Ports 1 wird das Einsatzteil 12 in den Steckabschnitt 111 eingesetzt derart, dass ein Vorsprung 121 des Einsatzteils 12 mit einer Aussparung 112 des Steckabschnitts 111 des unteren Gehäuseteils 11 in Eingriff gelangt und somit das Einsatzteil 12 verdrehsicher an dem unteren Gehäuseteil 11 gehalten ist. Im Bereich des Vorsprungs 121 ist ein Anschlussstück 122 in Form einer Kanüle angeordnet, das sich in montiertem Zustand des Ports durch die Aussparung 112 des unteren Gehäuseteils 11 hindurch erstreckt und einen Anschluss für den Katheter 2 schafft, über den eine Strömungsverbindung zwischen dem Katheter 2 und der Kammer 120 hergestellt werden kann. Über das Anschlussstück 122 kann somit ein Fluid aus der Kammer 120 in den Katheter 2 einfließen, wenn der Katheter 2 an das Anschlussstück 122 angeschlossen ist.

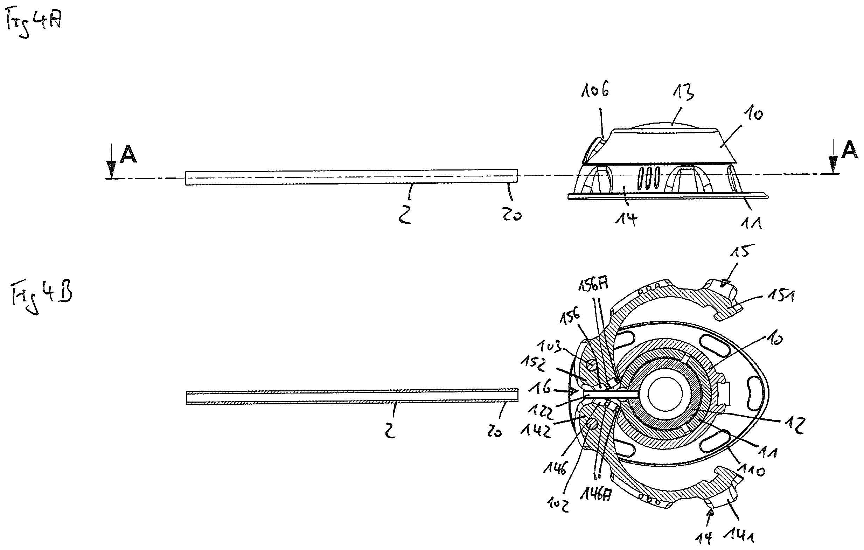

In montiertem Zustand des Ports 1 ist der Steckabschnitt 111 des unteren Gehäuseteils 11 in die Einfassung 101 des oberen Gehäuseteils 10 eingesteckt, wie dies beispielsweise aus Fig. 4B ersichtlich ist. Das Anschlussstück 122 in Form der Kanüle erstreckt sich dabei durch eine Aussparung 104 an der Einfassung 101 des oberen Gehäuseteils 10 hindurch und steht somit über die Einfassung 101 nach außen hin vor. Zwischen dem Einsatzteil 12 und einer oberen Umrandung der Öffnung 100 des oberen Gehäuseteils 10 kommt hierbei ein unterer Abschnitt 131 der Membran 13 zu liegen, so dass die Membran 13 klemmend zwischen dem Einsatzteil 12 und der Umrandung der Öffnung 100 gehalten ist.

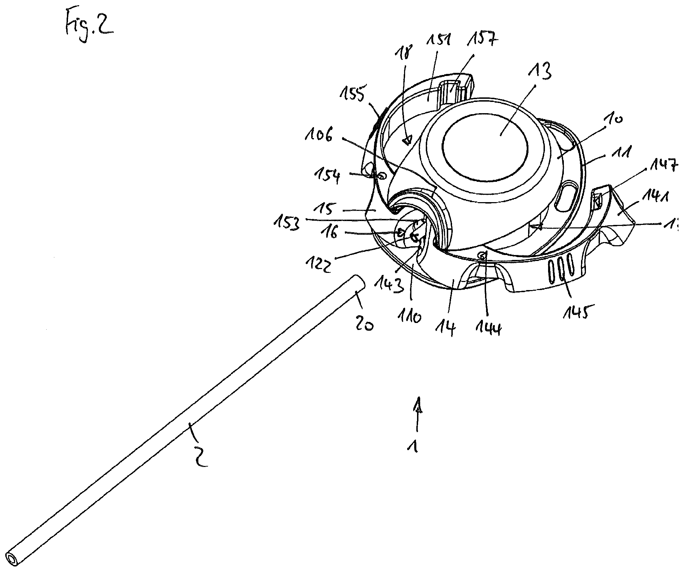

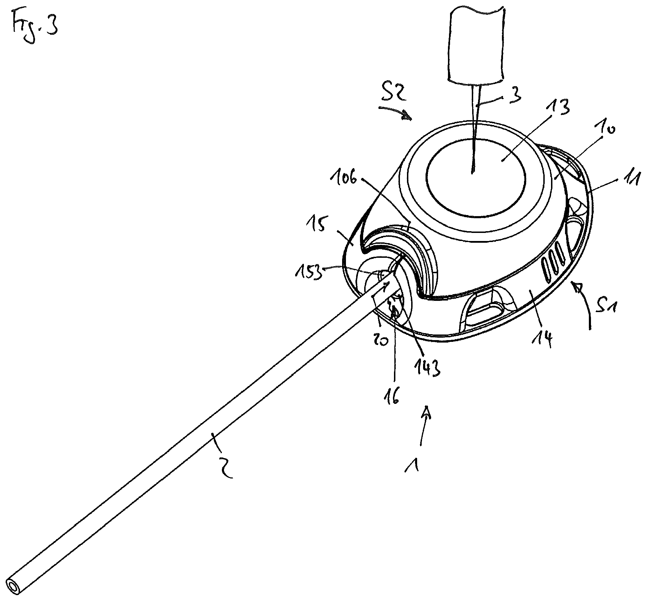

Der Port 1 weist zwei Hebel 14, 15 auf, die um Schwenkachsen 102, 103 an dem oberen Gehäuseteil 10 verschwenkbar gelagert sind und dazu dienen, den Katheter 2, wenn er an das Anschlussstück 122 angesetzt ist, zu arretieren, damit bei implantiertem Port 1 der Katheter 2 sich nicht von dem Anschlussstück 122 lösen kann. Die Hebel 14, 15 ermöglichen hierbei in einer ausgeschwenkten, ersten Stellung (siehe Fig. 2) ein Ansetzen des Katheters 2 an das Anschlussstück 122 und halten den Katheter 2 in einer eingeschwenkten, zweiten Stellung (siehe Fig. 3) klemmend an dem Anschlussstück 122.

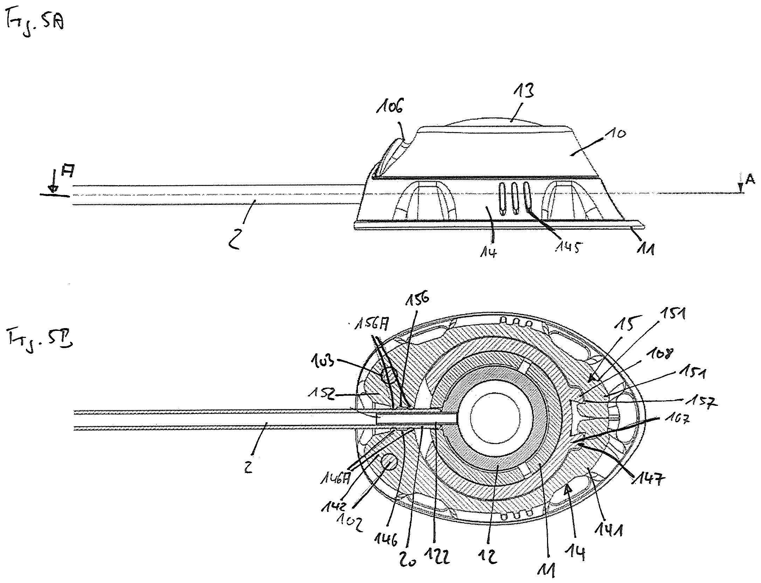

Die Hebel 14, 15 weisen jeweils zwei Hebelabschnitte 141, 142 bzw. 151, 152 auf. An dem längeren, ersten Hebelabschnitt 141, 151 ist eine Rasteinrichtung in Form von Rasthaken 147, 157 (siehe Fig. 5B) angeordnet, die in der eingeschwenkten, zweiten Stellung der Hebel 14, 15 mit Rastvorsprüngen 107, 108 am oberen Gehäuseteil 10 in Eingriff stehen und somit die Hebel 14, 15 mit dem oberen Gehäuseteil 10 verrasten. An dem kürzeren, zweiten Hebelabschnitt 142, 152 hingegen ist eine Klemmeinrichtung in Form eines Klemmabschnitts 146, 156 mit daran angeordneten Klemmrippen 146A, 156A (siehe Fig. 5B) angeordnet, der dazu dient, in der eingeschwenkten, zweiten Stellung der Hebel 14, 15 den Katheter 2 mit dem Anschlussstück 122 zu verklemmen, so dass der Katheter 2 nicht in unbeabsichtigter Weise von dem Anschlussstück 122 rutschen kann.

Dadurch, dass der erste Hebelabschnitt 141, 151 deutlich länger als der zweite Hebelabschnitt 142, 152 mit der daran angeordneten Klemmeinrichtung ausgebildet ist und ein Betätigen der Hebel 14, 15 über den ersten Hebelabschnitt 141, 151 mit daran angeordneten Griffrippen 145, 155 (siehe Fig. 2) erfolgt, können die Hebel 14, 15 zum Arretieren des Katheters 2 an dem Port 1 in leichter Weise manuell in eine zugeordnete Schwenkrichtung S1, S2 verschwenkt werden, wobei sich aufgrund der Hebelverhältnisse bei vergleichsweise kleiner Betätigungskraft eine vergleichsweise große Klemmkraft an dem zweiten Hebelabschnitt 142, 152 zum Verklemmen des Katheters 2 mit dem Anschlussstück 122 ergibt. Es ergibt sich eine einfache, haptisch angenehme Betätigung der Hebel 14, 15 zum Überführen der Hebel 14, 15 von der ausgeschwenkten, ersten Stellung (siehe Fig. 2) in die eingeschwenkte, zweite Stellung (siehe Fig. 3), die auch ohne weiteres durch einen Nutzer mit Operationshandschuhen durchgeführt werden kann.

Dadurch, dass zudem die Rasteinrichtung in Form der Rasthaken 147, 157 an dem von der Schwenkachse 102, 103 abliegenden Ende des ersten Hebelabschnitts 141, 151 angeordnet ist, muss die Rasteinrichtung nur eine vergleichsweise kleine Rastkraft aufbringen, um die Hebel 14, 15 zuverlässig in ihrer eingeschwenkten, zweiten Stellung zu halten. Darüber hinaus wird beim Überführen der Hebel 14, 15 von der ausgeschwenkten, ersten Stellung in die eingeschwenkte, zweite Stellung das Einschnappen der Rasthaken 147, 157 in formschlüssigen Eingriff mit den Rastvorsprüngen 107, 108 nicht gedämpft, so dass die Verrastung zum einen sicher einschnappt und zum zweiten ein deutlich hörbares Einschnappgeräusch erzeugt, was dem Nutzer eine Rückmeldung über die hergestellte Verrastung liefert.

Die Hebel 14, 15 weisen an ihren zweiten Hebelabschnitten 142, 152 jeweils eine Aussparung 143, 153 in Form einer Vertiefung auf, die zusammen eine Durchführungsöffnung 16 zum Ansetzen des Katheters 2 in der ausgeschwenkten, ersten Stellung der Hebel 14, 15 an das Anschlussstück 122 bereitstellen. In der ausgeschwenkten, ersten Stellung (siehe Fig. 2 sowie Fig. 4A, 4B) kann der Katheter 2 somit mit einem Ende 20 in die Durchführungsöffnung 16 eingeführt und auf das Anschlussstück 122 aufgeschoben werden, bis das Ende 20 des Katheters 2 an dem Einsatzteil 12 anliegt, wie dies in Fig. 5B dargestellt ist.

Zum Arretieren des Katheters 2 an dem Anschlussstück 122 werden dann die Hebel 14, 15 in Aufnahmeschlitze 17, 18, die zwischen der Bodenplatte 110 des unteren Gehäuseteils 11 und dem oberen Gehäuseteil 10 gebildet sind, eingeschwenkt, so dass die Hebel 14, 15 in ihre eingeschwenkte, zweite Stellung gelangen und die Rasthaken 147, 157 in Eingriff mit den Rastvorsprüngen 107, 108 am oberen Gehäuseteil 10 schnappen (siehe Fig. 3 sowie Fig. 5A, 5B).

Dadurch werden die Klemmabschnitte 146, 156 mit den daran angeordneten Klemmrippen 146A, 156A an den zweiten Hebelabschnitten 142, 152 der Hebel 14, 15 dem am Anschlussstück 122 angeordneten Katheter 2 angenähert und in klemmende Anlage mit dem Ende 20 des Katheters 2 gebracht, so dass der Katheter 2 an dem Anschlussstück 122 arretiert ist (siehe Fig. 5B). Der Katheter 2 ist somit fest an dem Anschlussstück 122 gehalten und kann nicht ohne weiteres, zumindest nicht in unbeabsichtigter Weise, von dem Anschlussstück 122 rutschen.

Um zu verhindern, dass die Hebel 14, 15 in unbeabsichtigter Weise bereits vor Ansetzen des Katheters 2 an das Anschlussstück 122 verschwenkt werden, ist an jedem Hebel 14, 15 eine Vertiefung 144, 154 angeordnet, die mit einem Druckpunkt 105, 106 an dem oberen Gehäuseteil 10 in Eingriff zu bringen ist. Beim Einschwenken der Hebel 14, 15 muss dieser Druckpunkt 105, 106 überwunden werden, was das Aufbringen einer gewissen Kraft erfordert und somit einem unbeabsichtigten, unbewussten Einschwenken der Hebel 14, 15 entgegenwirkt.

Die Hebel 14, 15 weisen, axial entlang der Schwenkachse 102, 103 versetzt zu dem Klemmabschnitt 146, 156, jeweils eine Verzahnung 149, 159 auf, die miteinander in Eingriff stehen und bewirken, dass die Hebel 14, 15 nur synchron miteinander verschwenkt werden können. Ein Verschwenken des einen Hebels 14, 15 ohne den anderen Hebel 15, 14 ist somit nicht möglich, was bewirkt, dass die Klemmabschnitte 146, 156 beim Einschwenken der Hebel 14, 15 in synchroner Weise klemmend in Anlage mit dem Katheter 2 gebracht werden. Dies ist in Fig. 7B dargestellt.

Wie ebenfalls aus Fig. 7B ersichtlich ist, weisen die Hebel 14, 15 im Bereich des zweiten Hebelabschnitts 142, 152 jeweils ein Langloch 148, 158 auf, in das ein Führungszapfen 116, 117 an der Bodenplatte 110 des unteren Gehäuseteils 11 eingreift. Mittels der in die Langlöcher 148, 158 eingreifenden Führungszapfen 116, 117 wird zum einen die Verschwenkbewegung der Hebel 14, 15 geführt. Zum anderen wird eine Endlage der Hebel 14, 15 insbesondere in der ausgeschwenkten, ersten Stellung definiert, so dass die Hebel 14, 15 nicht über die in Fig. 7B dargestellte Stellung hinaus ausgeschwenkt werden können.

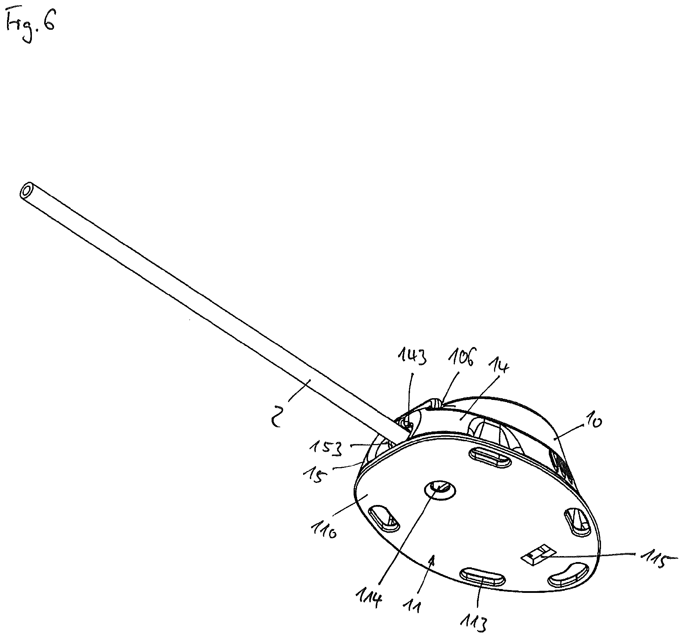

Wie aus der Ansicht schräg von unten gemäß Fig. 6 ersichtlich ist, sind an der Bodenplatte 110 des unteren Gehäuseteils 11 zum einen eine Sichtöffnung 114 und zum anderen eine Betätigungsöffnung 115 angeordnet. Über die Sichtöffnung 114 kann visuell überprüft werden, ob der Katheter 2 mit seinem Ende 20 richtig an das Anschlussstück 122 angesetzt ist. Kann visuell verifiziert werden, dass der Katheter 2 beispielsweise weit genug auf das Anschlussstück 112 aufgeschoben ist, so können die Hebel 14, 15 zum Arretieren des Katheters 2 an dem Port 1 eingeschwenkt werden. Die Betätigungsöffnung 115 dient dazu, die Verrastung der Hebel 14, 15 mit dem oberen Gehäuseteil 10 in der eingeschwenkten, zweiten Stellung gegebenenfalls wieder zu lösen, um den Katheter 2 von dem Port 1 entnehmen zu können. Hierzu kann ein Werkzeug in Form eines Formstücks durch die Betätigungsöffnung 115 eingeführt werden, um den Formschluss zwischen den Rasthaken 147, 157 und den zugeordneten Rastvorsprüngen 107, 108 aufzuheben und die Verrastung auf diese Weise zu lösen.

Zum Befüllen der Kammer 120 des Einsatzteils 12 wird die Membran 13 mittels einer Punktionskanüle 3 (siehe Fig. 3) durchstochen, und ein Wirkstoff wird in die Kammer 120 eingespritzt. Dies erfolgt in der Regel bei in einen Patienten implantiertem Port 1, so dass für den die Punktionskanüle 3 bedienenden Nutzer, beispielsweise eine Krankenschwester, die Membran 3 nicht frei sichtbar ist und der Port 1 mit seiner Membran 13 ertastet werden muss. Um hierbei das Risiko zu reduzieren, dass bei dem Versuch, die Membran 13 mit der Punktionskanüle 3 zu durchstechen, die Punktionskanüle 3 an dem oberen Gehäuseteil 10 abgleitet und den Katheter 2 punktiert, ist an dem oberen Gehäuseteil 10 an einer dem Katheter 2 zugewandten Seite der Membran 13 ein Ableitelement 106 vorgesehen, das nach Art einer Nut an das obere Gehäuseteil 10 angeformt ist, quer zu einer (gedachten) Verbindungslinie zwischen der Membran 13 und dem Katheter 2 erstreckt ist und die Punktionskanüle 3, sollte sie in Richtung des Katheters 2 rutschen, vom Katheter 2 wegleitet. Der Katheter 2 ist damit abgeschirmt.

Die Gehäuseteile 10, 11 des Ports 1 können in vorteilhafter Weise aus einem biokompatiblen Material, beispielsweise einem biokompatiblen Kunststoff, hergestellt werden. Das Einsatzteil 12 hingegen kann aus einem für den Wirkstoff resistenten Material, beispielsweise einem metallischen Material oder einem keramischem Material, bestehen. Ein solches Material ist gegenüber verschiedensten Wirkstoffen resistent und weist zudem eine hinreichende Festigkeit auf, so dass das Einsatzteil 12 nicht von einer Punktionskanüle 3 durchstochen werden kann.

Der der Erfindung zugrunde liegende Gedanke ist nicht auf die vorangehend geschilderten Ausführungsbeispiele beschränkt, sondern lässt sich auch in gänzlich anders gearteter Weise verwirklichen.

Insbesondere ist nicht zwingend erforderlich, dass der Port die beschriebene dreiteilige Bauform mit einem oberen Gehäuseteil, einem unteren Gehäuseteil und einem davon eingefassten Einsatzteil aufweist. Grundsätzlich sind auch andere Bauformen, die beispielsweise kein separates Einsatzteil verwenden, denkbar und möglich.

In einer alternativen Ausführungsform (nicht dargestellt) verrasten die Hebel 14, 15 nicht mit dem Gehäuseteil 10, sondern mit sich selber, beispielsweise dadurch, dass an dem einen Haken ein Rastvorsprung und an dem anderen Hebel ein Rasthaken ausgebildet ist. Die Fixierung mit dem Gehäuse, insbesondere dem Gehäuseteil 10, 11, kann in diesem Falle beispielsweise über einen Formschluss und/oder Kraftschluss erreicht werden. Ebenfalls ist es möglich, dass eine weitere Rastvorrichtung an zumindest einem Hebel 14, 15 und dem Gehäuseteil 10, 11 vorgesehen ist, so dass neben einer Verrastung der beiden Hebel 14, 15 auch eine Verrastung mit dem Gehäuseteil 10, 11 erfolgt. Die betreffs der Verrastung der Hebel 14, 15 mit dem Gehäuseteil 10 genannten Vorteile liegen bei dieser Ausführungsform natürlich ebenfalls vor.

In einer weiteren alternativen Ausführungsform (nicht dargestellt) umfasst der Hebel 14,15 keine Vertiefung 144, 145, der Druckpunkt 105 wird lediglich überwunden, ohne dass ein Eingriff des Druckpunkts 105 in einer Vertiefung des Hebels 14, 15 stattfindet.

### Bezugszeichenliste

- 1: Port
- 10: Gehäuseteil
- 100: Öffnung
- 101: Einfassung
- 102, 103: Schwenkachse
- 104: Aussparung
- 105: Druckpunkt
- 106: Ableitelement (Prallkante)
- 11: Gehäuseteil
- 110: Bodenplatte
- 111: Steckabschnitt
- 112: Aussparung
- 113: Nahtlöcher
- 114: Sichtöffnung
- 115: Betätigungsöffnung
- 116, 117: Führungszapfen
- 12: Einsatzteil
- 120: Kammer
- 121: Vorsprung
- 122: Anschlussstück (Kanüle)
- 13: Membran
- 130: Kopfabschnitt
- 131: Unterer Abschnitt
- 14, 15: Hebel
- 140, 150: Lageröffnung
- 141, 151: Hebelabschnitt
- 142, 152: Hebelabschnitt
- 143, 153: Aussparung
- 144, 154: Vertiefung
- 145, 155: Griffrippen
- 146, 156: Klemmabschnitt
- 146A, 156A: Klemmrippen
- 147, 157: Rasthaken
- 148, 158: Langloch
- 149, 159: Verzahnung
- 16: Durchführungsöffnung
- 2: Katheter
- 20: Ende
- 3: Punktionskanüle

## Patentansprüche

1. Port (1) für einen Katheter (2), mit
- einem Gehäuse (10, 11),
- einer in dem Gehäuse (10, 11) angeordneten Kammer (120) zum Aufnehmen eines medizinischen Wirkstoffs,
- einem Anschlussstück (122) zum Anschließen eines Katheters (2), um eine Strömungsverbindung zwischen der Kammer (120) und dem Katheter (2) herzustellen, und
- mindestens einem Hebel (14, 15), der um eine Schwenkachse (102, 103) des Gehäuses (10, 11) schwenkbar gelagert ist, wobei der mindestens eine Hebel (14, 15) ausgebildet ist, in einer ersten Schwenkstellung ein Ansetzen des Katheters (2) an das Anschlussstück (122) zuzulassen und in einer zweiten Schwenkstellung den Katheter (2) klemmend an dem Anschlussstück (122) zu halten,
**dadurch gekennzeichnet,**
**dass** der mindestens eine Hebel (14, 15) einen ersten Hebelabschnitt (141, 151) und einen von dem ersten Hebelabschnitt (141, 151) unterschiedlichen, zweiten Hebelabschnitt (142, 152) aufweist, wobei der erste Hebelabschnitt (141, 151) eine Rasteinrichtung (147, 157) zum Verrasten des mindestens einen Hebels (14, 15) mit dem Gehäuse (10, 11) und/oder einem ersten Hebelabschnitt (141, 151) eines weiteren Hebels (14, 15) in der zweiten Schwenkstellung und der zweite Hebelabschnitt (142, 152) eine Klemmeinrichtung (146, 156) zum klemmenden Halten des Katheters (2) in der zweiten Schwenkstellung trägt.

2. Port (1) nach Anspruch 1, **dadurch gekennzeichnet, dass** die Klemmeinrichtung einen Klemmabschnitt (146, 157) umfasst, der in der ersten Schwenkstellung derart von dem Anschlussstück (122) entfernt ist, dass der Katheter (2) an das Anschlussstück (122) ansetzbar ist, und in der zweiten Schwenkstellung dem Anschlussstück (122) im Vergleich zur ersten Schwenkstellung zum klemmenden Halten des Katheters (2) an dem Anschlussstück (122) angenähert ist.

3. Port (1) nach Anspruch 1 oder 2, **dadurch gekennzeichnet, dass** der zweite Hebelabschnitt (142, 152) kürzer als der erste Hebelabschnitt (141, 151) ist.

4. Port (1) nach einem der Ansprüche 1 bis 3, **dadurch gekennzeichnet, dass** der erste Hebelabschnitt (141, 151) und der zweite Hebelabschnitt (142, 152) sich in unterschiedliche Richtungen von der Schwenkachse (102, 103) erstrecken.

5. Port (1) nach einem der vorangehenden Ansprüche, **dadurch gekennzeichnet, dass** an dem Klemmabschnitt (146, 156) mindestens eine Klemmrippe (146A, 156A) angeordnet ist, die quer zu einer Schwenkrichtung (S1, S2), entlang derer der mindestens eine Hebel (14, 15) schwenkbar ist, erstreckt ist.

6. Port (1) nach einem der vorangehenden Ansprüche, **dadurch gekennzeichnet, dass** die Rasteinrichtung (147, 157) durch einen Rasthaken ausgebildet ist, der in der zweiten Schwenkstellung rastend mit einem zugeordneten Rastvorsprung (107, 108) des Gehäuses (10, 11) und/oder eines weiteren Hebels (14, 15) in Eingriff steht.

7. Port (1) nach einem der vorangehenden Ansprüche, **dadurch gekennzeichnet, dass** die Verrastung des mindestens einen Hebels (14, 15) mit dem Gehäuse (10, 11) und/oder mit dem weiteren Hebel (14, 15) in der zweiten Schwenkstellung lösbar ist.

8. Port (1) nach Anspruch 7, **dadurch gekennzeichnet, dass** das Gehäuse (10, 11) zum Lösen der Verrastung eine Betätigungsöffnung (115) aufweist, durch die hindurch die Rasteinrichtung (147, 157) zum Lösen betätigbar ist.

9. Port (1) nach einem der vorangehenden Ansprüche, **gekennzeichnet durch** zwei Hebel (14, 15), die jeweils einen ersten Hebelabschnitt (141, 151) und einen von dem ersten Hebelabschnitt (141, 151) unterschiedlichen, zweiten Hebelabschnitt (142, 152) aufweisen.

10. Port (1) nach Anspruch 9, **dadurch gekennzeichnet, dass** die Hebel (14, 15) um gleichgerichtete Schwenkachsen (102, 103) verschwenkbar sind.

11. Port (1) nach Anspruch 9 oder 10, **dadurch gekennzeichnet, dass** die Hebel (14, 15) in der ersten Schwenkstellung zwischen ihren zweiten Hebelabschnitten (142, 152) eine Durchführungsöffnung (16) ausbilden, durch die hindurch der Katheter (2) führbar ist.

12. Port (1) nach einem der Ansprüche 9 bis 11, **dadurch gekennzeichnet, dass** die Hebel (14, 15) jeweils an dem zweiten Hebelabschnitt (142, 152) eine Verzahnung (149,159) aufweisen und die Verzahnungen (149, 159) der Hebel (14, 15) miteinander in Eingriff stehen derart, dass bei einem Verschwenken des einen Hebels (14, 15) auch der andere Hebel (15, 14) verschwenkt wird.

13. Port (1) nach einem der vorangehenden Ansprüche, **dadurch gekennzeichnet, dass** an dem Gehäuse (10, 11) mindestens ein Druckpunkt (105) angeordnet ist, der beim Überführen des mindestens einen Hebels (14, 15) von der ersten Schwenkstellung in die zweite Schwenkstellung zu überwinden ist, alternativ mit einer Vertiefung (144, 154) an dem mindestens einen Hebel (14, 15) in Eingriff zu bringen ist.

14. Port (1) nach einem der vorangehenden Ansprüche, **dadurch gekennzeichnet, dass** das Gehäuse (10, 11) eine Sichtöffnung (114) aufweist, durch die hindurch die Anordnung des Katheters (2) an dem Anschlussstück (122) in der zweiten Schwenkstellung prüfbar ist.

15. Port (1) nach einem der vorangehenden Ansprüche, **dadurch gekennzeichnet, dass** der Port (1) eine an dem Gehäuse (10, 11) gehaltene Membran (13) und ein Ableitelement (106) aufweist, wobei das Ableitelement (106) an dem Gehäuse (10, 11) an einer hin zu dem Anschlussstück (122) weisenden Seite der Membran (13) angeordnet und ausgebildet ist, ein Abrutschen einer Punktionskanüle (3) hin zu dem an das Anschlussstück (122) des Ports (1) angeschlossenen Katheter (2) zu verhindern.

16. System mit
- einem Port (1) nach einem der vorangehenden Ansprüche und
- einem Katheter (2), der in der ersten Schwenkstellung des mindestens einen Hebels (14, 15) klemmend an dem Anschlussstück (122) des Ports (1) gehalten ist und in der zweiten Schwenkstellung des mindestens einen Hebels (14, 15) an das Anschlussstück (122) ansetzbar ist.

## Claims

1. Port (1) for a catheter (2), having
- a housing (10, 11),
- a chamber (120) arranged in the housing (10, 11) for receiving a medically active substance,
- a connecting piece (122) for connecting a catheter (2) in order to produce a flow connection between the chamber (120) and the catheter (2) and
- at least one lever (14, 15) which is mounted so as to be pivotable about a pivot axis (102, 103) of the housing (10, 11), wherein the at least one lever (14, 15) is realized to allow the catheter (2) to be fitted onto the connecting piece (122) in a first pivot position and to hold the catheter (2) in a clamping manner on the connecting piece (122) in a second pivot position,
**characterized in that** the at least one lever (14, 15) comprises a first lever portion (141, 151) and a second lever portion (142, 152) which is different to the first lever portion (141, 151), wherein the first lever portion (141, 151) carries a latching device (147, 157) for latching the at least one lever (14, 15) with the housing (10, 11) and/or with a first lever portion (141, 151) of a further lever (14, 15) in the second pivot position and the second lever portion (142, 152) carries a clamping device (146, 156) for holding the catheter (2) in a clamping manner in the second pivot position.

2. Port (1) according to Claim 1, **characterized in that** the clamping device includes a clamping portion (146, 157) which is removed from the connecting piece (122) in the first pivot position in such a manner that the catheter (2) is fittable onto the connecting piece (122), and in the second pivot position is moved closer to the connecting piece (122), in comparison to the first pivot position, for holding the catheter (2) in a clamping manner on the connecting piece (122).

3. Port (1) according to Claim 1 or 2, **characterized in that** the second lever portion (142, 152) is shorter than the first lever portion (141, 151).

4. Port (1) according to one of Claims 1 to 3, **characterized in that** the first lever portion (141, 151) and the second lever portion (142, 152) extend in different directions from the pivot axis (102, 103).

5. Port (1) according to one of the preceding claims, **characterized in that** at least one clamping rib (146A, 156A), which extends transversely with respect to a pivoting direction (S1, S2), along which the at least one lever (14, 15) is pivotable, is arranged on the clamping portion (146, 156).

6. Port (1) according to one of the preceding claims, **characterized in that** the latching device (147, 157) is realized by a latching hook which engages in a latching manner with an associated latching projection (107, 108) of the housing (10, 11) and/or of a further lever (14, 15) in the second pivot position.

7. Port (1) according to one of the preceding claims, **characterized in that** the latching of the at least one lever (14, 15) with the housing (10, 11) and/or with the further lever (14, 15) is releasable in the second pivot position.

8. Port (1) according to Claim 7, **characterized in that** for releasing the latching arrangement, the housing (10, 11) comprises an actuating opening (115) through which the latching device (147, 157) is actuatable for release.

9. Port (1) according to one of the preceding claims, **characterized by** two levers (14, 15) which comprise in each case a first lever portion (141, 151) and a second lever portion (142, 152) which is different to the first lever portion (141, 151).

10. Port (1) according to Claim 9, **characterized in that** the levers (14, 15) are pivotable about pivot axes (102, 103) which are aligned in the same manner.

11. Port (1) according to Claim 9 or 10, **characterized in that** a through-opening (16), through which the catheter (2) is guidable, is realized by the levers (14, 15) between their second lever portions (142, 152) in the first pivot position.

12. Port (1) according to one of Claims 9 to 11, **characterized in that** the levers (14, 15) comprise in each case a toothing (149, 159) on the second lever portion (142, 152) and the toothings (149, 159) of the levers (14, 15) engage together in such a manner that when the one lever (14, 15) is pivoted, the other lever (14, 15) is also pivoted.

13. Port (1) according to one of the preceding claims, **characterized in that** at least one pressure point (105), which has to be overcome when transferring the at least one lever (14, 15) from the first pivot position into the second pivot position, as an alternative to this has to be moved into engagement with an indentation (144, 154) on the at least one lever (14, 15), is arranged on the housing (10, 11).

14. Port (1) according to one of the preceding claims, **characterized in that** the housing (10, 11) comprises an inspection opening (114) through which the arrangement of the catheter (2) on the connecting piece (122) in the second pivot position is checkable.

15. Port (1) according to one of the preceding claims, **characterized in that** the port (1) comprises a membrane (13) which is held on the housing (10, 11) and a deflecting element (106), wherein the deflecting element (106) is arranged and realized on the housing (10, 11) on a side of the membrane (13) which points toward the connecting piece (122) in order to prevent a puncturing cannula (3) from slipping toward the catheter (2) which is connected to the connecting piece (122) of the port (1).

16. System having
- a port (1) according to one of the preceding claims and
- a catheter (2) which is held in a clamping manner on the connecting piece (122) of the port (1) in the first pivot position of the at least one lever (14, 15) and is fittable onto the connecting piece (122) in the second pivot position of the at least one lever (14, 15).

## Revendications

1. Port (1) pour un cathéter (2), avec :
- un boîtier (10, 11) ;
- une chambre (120) disposée dans le boîtier (10, 11) servant à loger une substance active médicale ;
- un raccord (122) pour raccorder un cathéter (2), en vue d'établir une liaison d'écoulement entre la chambre (120) et le cathéter (2) ; et
- au moins un levier (14, 15) disposé de façon à pouvoir pivoter autour d'un axe de pivotement (102, 103) du boîtier (10, 11), l'au moins un levier (14, 15) étant réalisé pour permettre, dans une première position de pivotement, un placement du cathéter (2) contre le raccord (122) et pour maintenir dans une deuxième position de pivotement le cathéter (2) en le coinçant contre le raccord (122) ;
**caractérisé en ce que** :
l'au moins un levier (14, 15) comporte une première section de levier (141, 151) et une deuxième section de levier (142, 152) différente de la première section de levier (141, 151), la première section de levier (141, 151) ayant un dispositif d'encliquetage (147, 157) pour encliqueter l'au moins un levier (14, 15) avec le boîtier (10, 11) et/ou avec une première section de levier (141, 151) d'un levier (14, 15) supplémentaire dans la deuxième position de pivotement et la deuxième section de levier (142, 152) ayant un dispositif de coincement (146, 156) pour maintenir de façon coincée le cathéter (2) dans la deuxième position de pivotement.

2. Port (1) selon la revendication 1, **caractérisé en ce que** le dispositif de coincement comprend une section de coincement (146, 157) éloignée de telle sorte dans la première position de pivotement du raccord (122) que le cathéter (2) peut être placé contre le raccord (122) et rapproché, dans la deuxième position de pivotement, du raccord (122), par rapport à la première position de pivotement, pour maintenir de façon coincée le cathéter (2) contre le raccord (122).

3. Port (1) selon la revendication 1 ou 2, **caractérisé en ce que** la deuxième section de levier (142, 152) est plus courte que la première section de levier (141, 151).

4. Port (1) selon l'une quelconque des revendications 1 à 3, **caractérisé en ce que** la première section de levier (141, 151) et la deuxième section de levier (142, 152) s'étendent dans des directions différentes par rapport à l'axe de pivotement (102, 103).

5. Port (1) selon l'une quelconque des revendications précédentes, **caractérisé en ce qu'**au moins une nervure de coincement (146A, 156A) est disposée au niveau de la section de coincement (146, 156), cette nervure s'étendant transversalement à une direction de pivotement (S1, S2) le long de laquelle l'au moins un levier (14, 15) peut pivoter.

6. Port (1) selon l'une quelconque des revendications précédentes, **caractérisé en ce que** le dispositif d'encliquetage (147, 157) est réalisé par le biais d'un crochet d'encliquetage en prise de façon encliquetée, dans la deuxième position de pivotement, avec une saillie d'encliquetage (107, 108) associée du boîtier (10, 11) et/ou d'un levier (14, 15) supplémentaire.

7. Port (1) selon l'une quelconque des revendications précédentes, **caractérisé en ce que** l'encliquetage de l'au moins un levier (14, 15) avec le boîtier (10, 11) et/ou avec le levier (14, 15) supplémentaire peut être défait dans la deuxième position de pivotement.

8. Port (1) selon la revendication 7, **caractérisé en ce que** le boîtier (10, 11) comporte une ouverture d'actionnement (115) pour libérer l'encliquetage et à travers laquelle le dispositif d'encliquetage (147, 157) peut être actionné pour le desserrage.

9. Port (1) selon l'une quelconque des revendications précédentes, **caractérisé par** la présence de deux leviers (14, 15), comportant respectivement une première section de levier (141, 151) et une deuxième section de levier (142, 152) différente de la première section de levier (141, 151).

10. Port (1) selon la revendication 9, **caractérisé en ce que** les leviers (14, 15) peuvent pivoter autour d'axes de pivotement (102, 103) de même orientation.

11. Port (1) selon la revendication 9 ou 10, **caractérisé en ce que** les leviers (14, 15) forment dans la première position de pivotement, entre leurs deuxièmes sections de levier (142, 152), une ouverture traversante (16) à travers laquelle le cathéter (2) peut être conduit.

12. Port (1) selon l'une quelconque des revendications 9 à 11, **caractérisé en ce que** les leviers (14, 15) comportent respectivement au niveau de la deuxième section de levier (142, 152) un endentement (149, 159) et que les endentements (149, 159) des leviers (14, 15) s'engrènent entre eux de telle sorte qu'en cas de pivotement du premier levier (14, 15), l'autre levier (15, 14) est lui aussi pivoté.

13. Port (1) selon l'une quelconque des revendications précédentes, **caractérisé en ce qu'**au moins un point de pression (105) est disposé au niveau du boîtier (10, 11), ce point de pression devant être dépassé lors du passage de l'au moins un levier (14, 15) de la première position de pivotement dans la deuxième position de pivotement et étant amené en prise en variante avec un renfoncement (144, 154) au niveau de l'au moins un levier (14, 15).

14. Port (1) selon l'une quelconque des revendications précédentes, **caractérisé en ce que** le boîtier (10, 11) comporte une ouverture de regard (114) permettant de contrôler le placement du cathéter (2) contre le raccord (122) dans la deuxième position de pivotement.

15. Port (1) selon l'une quelconque des revendications précédentes, **caractérisé en ce que** le port (1) comporte une membrane (13) maintenue contre le boîtier (10, 11) et un élément de dérivation (106), l'élément de dérivation (106) étant disposé et réalisé de façon à éviter, au niveau du boîtier (10, 11), au niveau d'un côté de la membrane (13) orienté vers le raccord (122), un glissement d'une canule de ponction (3) en direction du cathéter (2) raccordé au raccord (122) du port (1).

16. Système avec :
- un port (1) selon l'une quelconque des revendications précédentes ; et
- un cathéter (2) maintenu, dans la première position de pivotement de l'au moins un levier (14, 15), de façon à être coincé contre le raccord (122) du port (1) et pouvant être placé, dans la deuxième position de pivotement de l'au moins un levier (14, 15), contre le raccord (122).
